# EUROPEAN PATENT SPECIFICATION

(11) **EP 1 060 639 B1**
(45) Date of publication and mention of the grant of the patent: **08.03.2006**
(21) Application number: 98966744.9
(22) Date of filing: 18.12.1998
(51) Int. Cl.: H04R 25/00

(54) **COMPLIANT HEARING AID AND METHOD OF MANUFACTURE**
BIEGSAMES HÖRHILFEGERÄT UND VERFAHREN ZUR HERSTELLUNG
PROTHESE AUDITIVE SOUPLE

(30) Priority: 18.12.1997 US 68035 P; 26.05.1998 US 84864; 28.10.1998 US 181539; 28.10.1998 US 181540; 28.10.1998 US 181541; 28.10.1998 US 181842; 28.10.1998 US 181843; 28.10.1998 US 181844; 28.10.1998 US 181845
(43) Date of publication of application: 20.12.2000
(73) Proprietor: Softear Technologies, L.L.C., Harahan, LA 70123 (US)
(72) Inventor: JUNEAU, Roger, P., Destrehan, LA 70047 (US); CREEL, Lynn, P., Kenner, LA 70065 (US); DESPORTE, Edward, J., Covington, LA 70433 (US); MAJOR, Michael, W., Kenner, LA 70065 (US); SIEGLE, Gregory, R., Kenner, LA 70065 (US); KINLER, Kelly, M., Luling, LA 70070 (US)
(74) Representative: Harrison Goddard Foote
(86) International application number: PCT/US1998/026973
(87) International publication number: WO 1999/031934

(56) References cited:
- WO-A-92/03894
- WO-A-93/25053
- JP-A- 61 238 198
- US-A- 4 375 016
- US-A- 4 729 451

## Description

### CROSS-REFERENCE TO RELATED APPLICATIONS

In the US, this is a continuation-in-part of our co-pending US Patent Application Serial Nos. 09/181,539, 09/181,540, 09/181/541, 09/181/842, 09/181/843, 09/181/844, and 09/181/845, all filed 28 October 1998, which are continuations-in-part of our co-pending US Patent Application Serial No. 09/084,864, filed 26 May 1998. Priority of these applications is hereby claimed.

Priority of US Provisional Patent Application Serial No. 60/068,035, filed 18 December 1997, is hereby claimed.

### BACKGROUND OF THE INVENTION

### 1. Field of the Invention

The present invention relates to hearing aids and more particularly to an improved hearing aid, its method of manufacture and an improved method of compensating for hearing loss. More particularly, the present invention provides an improved method and apparatus for compensating for hearing loss that uses a construction combining a rigid mounting member (for example, a face plate) with a soft polymeric body that is joined to the mounting member and which encapsulates some of the electronic hearing aid components of the apparatus, the soft polymeric body being sized and shaped to conform to the user's ear canal during use. It may be possible to use a soft polymeric material as the face plate.

### 2. General Background of the Invention

The hearing industry has realized major strides in the development of high-fidelity, high-performance products, the most recent of which is digital signal processing technology. Hearing care professionals expected those advancements to solve the shortcomings of traditional amplification, and to push the market forward. Those expectations have not been fully realized. While these developments have solved many of the problems associated with traditional electronic design and steadily gained market share, they have not fostered overall market growth.

The issues of early acoustic feedback, less than optimum fidelity and intermodulation of the frequency response cannot be completely resolved by electronic manipulation of the signal by either analog or digital means.

Historically, custom-molded ear worn hearing instruments have been limited to an "acrylic pour" process as the means of the construction. With the advent of miniaturization and technological advancement of computer chip programming, the ear-wom instruments have become smaller and are positioned into the bony portion of the ear canal, commonly referred to as "deep insertion technology".

Developments outside the hearing industry have culminated in a new level of micro-miniaturization of electronic components for industry applications. Consequently, advanced signal processing can be housed in less space than was required for traditional electro-acoustic components.

With the development of programmable hearing aids, using either analog or digital signal processing, custom electronic design has shifted from the manufacturing level to the clinical level. The clinician can now customize the electro-acoustic response via software. It is no longer necessary for the device to be returned to the manufacturer for hardware changes to arrive at the desired electro-acoustic response. However, it is still often necessary to return the device for shell modifications.

In direct contrast to electronic advances within the industry, little or no advancement has been realized in custom prosthetic design. Since the late 1960's, when the custom in-the-ear hearing aid was developed, materials and construction techniques remained virtually unchanged. These materials and techniques were adopted from the dental industry, whereby the customized housing-commonly called a "shell" was constructed using acrylic of 90 point Durometer Hardness Shore D. This construction process provided the structure and the strength of material necessary to protect the electronics.

At the time the acrylic shell was developed, hearing instruments were worn in the relatively forgiving cartilaginous portion of the ear canal. Micro-miniaturization of electronic components, combined with increased consumer demand for a cosmetically acceptable device, has shifted the placement of the hearing aid toward the bony portion of the ear canal.

The bony portion of the canal is extremely sensitive and intolerant of an acrylic shell when that shell is over sized due to standard waxing procedures or is in contact with the canal wall beyond the second anatomical bend. Rigid acrylic that does not compress must pivot in reaction to jaw or head movement, thereby changing the direction of the receiver yielding a distorted acoustic response. In addition, the pivot action causes displacement of the device resulting in unwanted acoustic feedback. This problem has necessitated countless shell modifications, thereby compromising the precision approach of the original dental technology. Many such devices require some modification by the manufacturer. Most manufacturers can expect a high percentage of returns for modification or repair within the first year. Consequently, CIC (completely in canal) shell design has been reduced to more of a craft than a science. Although the recent introduction of the ultra-violet curing process has produced a stronger, thinner shell, the overall Shore Hardness remained unchanged.

The current trend for custom hearing aid placement is to position the instrument toward the bony portion of the ear canal. The ear canal can be defined as the area extending from the concha to the tympanic membrane. It is important to note that the structure of this canal consists of elastic cartilage laterally, and porous bone medially. The cartilaginous portion constitutes the outer one third of the ear canal. The medial two-thirds of the ear canal is osseous or bony. The skin of the osseous canal, measuring only about 0.2 mm in thickness, is much thinner than that of the cartilaginous canal, which is 0.5 to 1 mm in thickness. The difference in thickness directly corresponds to the presence of apocrine (ceruminous) and sebaceous glands found only in the fibrocartilaginous area of the canal. Thus, this thin-skinned thinly-lined area of the bony canal is extremely sensitive to any hard foreign body, such as an acrylic hearing instrument.

Exacerbating the issue of placement of a hard foreign body into the osseous area of the ear canal is the ear canal's dynamic nature. It is geometrically altered by temporomandibular joint action and by changes in head position. This causes elliptical elongation (widening) of the ear canal. These alterations in canal shape vary widely from person to person. Canal motion makes it very difficult to achieve a comfortable, true acoustic seal with hard acrylic material. When the instrument is displaced by mandibular motion, a leakage or "slit leak" creates an open loop between the receiver and the microphone and relates directly to an electroacoustic distortion commonly known as feedback. Peripheral acoustic leakage is a complex resonator made up of many transient resonant cavities. These cavities are transient because they change with jaw motion as a function of time, resulting in impedance changes in the ear canal. These transients compromise the electroacoustic performance.

The properties of hard acrylic have limitations that require modification to the hard shell exterior to accommodate anatomical variants and the dynamic nature of the ear canal. The shell must be buffed and polished until comfort is acceptable. The peripheral acoustic leakage caused by these modifications results in acoustic feedback before sufficient amplification can be attained.

Hollow shells used in today's hearing aid designs create internal or mechanical feedback pathways unique to each device. The resulting feedback requires electronic modifications to "tweak" the product to a compromised performance or a "pseudo-perfection". With the industry's efforts to facilitate the fine-tuning of hearing instruments for desired acoustic performance, programmable devices were developed. The intent was to reduce the degree of compromise, but by their improved frequency spectrum the incidence of feedback was heightened. As a result, the industry still falls well short of an audiological optimum.

A few manufacturers have attempted all-soft, hollow shells as alternatives to acrylic, hollow shells. Unfortunately, soft vinyl materials shrink, discolor, and harden after a relatively short period of wear. Polyurethane has proven to provide a better acoustic seal than polyvinyl, but has an even shorter wear life (approximately three months). Silicones have a long wear life but are difficult to bond with plastics such as acrylic, a necessary process for the construction of custom hearing instruments. To date, acrylic has proven to be the only material with long term structural integrity. The fact remains, however, that the entire ear is a dynamic acoustic environment and is ill-served by a rigid material such as acrylic. Also, the acrylic hearing aids typically need to be returned to the manufacturer for major shell modifications.

The following references are made :
U.S. Patent Nos.: 4,051,330; 4,375,016; 4,607,720; 4,716,985; 4,729,451; 4,811,402; 4,870,688; 4,880,076; 4,937,876; 5,002,151; 5,068,902; 5,185,802; 5,201,007; 5,259,032; 5,530,763; 5,430,801; 5,500,902; and 5,659,621. International Patent Application Nos: WO93/25053 and WO92/03894.

Also of interest are published Japanese patent application no. JA61-238198, the articles from December 1997 Journal of American Academy of Audiology, and Staab, Wayne J. and Barry Finlay, "A fitting rationale for deep fitting canal hearing instruments", Hearing Instruments, Vol. 42, No. 1, 1991, pp. 7-10, 48.

### BRIEF SUMMARY OF THE INVENTION

The present invention provides a method and material for the construction of a soft hearing instrument that is solid (i.e. eliminates void spaces). This instrument includes a soft body portion that is truly soft, comprising an elastomer of about 3 to 55 durometer Shore A and preferably 10-35 durometer Shore A. This product is unique in that it is solid, with the electronic components actually encapsulated or embedded within the soft fill material. The fill material can be a Dow Corning® MDX-4-4210 silicone or a silicone polymer distributed by Factor II, Inc. of Lakeside, Arizona, designated as product name 588A, 588B, 588V.

The present invention provides a method that can replace traditional acrylic shell construction. Unlike the shell construction process, the ear impression is not modified, built up, or waxed. With the elimination of these steps, a more faithful reproduction of the ear impression is accomplished. With the present invention, the manufacturer should be able to produce a hearing aid body which will not need to be returned as frequently for modification as with present hard acrylic hearing aid bodies.

The apparatus of the present invention is virtually impervious to the discoloration, cracking, and hardening experienced with polyvinyls and polyurethanes.

The hearing aid of the present invention provides a greater range of gain before feedback occurs.

The outer surface of the body of the present invention is preferably non-absorbent and virtually impervious to cerumen.

As used herein, "in the ear hearing aids" includes all hearing aids which have all of the electronics positioned in the ear, and thus includes hearing aid styles ranging from full concha to CIC (completely in the canal) hearing aid styles.

The preferred embodiment of the present invention shown in the drawings is a CIC hearing aid style.

The present invention is more fully described in the claims as filed in the parent patent applications and in the present application as filed, all of which claims are incorporated by reference.

According to a first aspect, the present invention provides an in the ear hearing aid comprising:
a plate member with electronic hearing aid components mounted thereto; and
a soft polymeric body that is bonded to the plate member, which encapsulates at least some of the electronic hearing aid components, and is shaped to conform to the ear canal of a user; "whereby the soft polymeric body and encapsulated electronic hearing aid components define a soft structure that is compliant to the ear canal during use, is substantially solid and free of void spaces between at least some of the components and the ear canal"; and wherein the combination of the soft polymeric body and encapsulated electronic hearing aid components minimises feedback.

Preferably, the soft polymeric body includes silicone, and advantageously has a hardness of between 3 and 55 Durometer Shore A or between 10 and 35 Durometer Shore A. The plate member may be acrylic, and the soft polymeric body may anatomically fit the contours of the ear canal. The plate member may be generally circular.

Both of the medial and lateral sides of the mounting member may carry at least one electronic hearing aid component. The bonding layer may comprise in part a bonding enhancer. The mounting member may be acrylic at the medial surface, and the bonding layer may include a bonding enhance that coats the medial surface. The soft polymeric body may include silicone. The mounting member may be harder than the soft polymeric body. The bonding layer may comprise multiple layers at least one of which may be a bonding enhancer.

The hearing aid may be sized to fit completely in the ear canal of a user.

According to a second aspect, the present invention provides a method of manufacturing a hearing aid comprising the steps of:
a) forming a hollow shell with an inside surface that approximates the shape of the human ear canal, the shell being of a soft polymeric material;
b) providing a mounting member;
c) mounting electronic hearing aid components to the mounting member;
d) temporarily joining the hollow shell to the mounting member to define a mold cavity;
e) filling the mold cavity with a soft polymeric material that substantially encapsulates the electronic components;
f) eliminating substantially all void space between the shell and the electronic components with the filling in step "e";
g) allowing the soft polymeric material to cure;
h) removing the shell;
i) wherein in step "e" the combination of electronic components and fill material define a soft structure that is compliant to ear canal movement during use.

The hearing aid of the aforementioned method may be sized to fit completely in the ear canal of the patient.

### BRIEF DESCRIPTION OF THE DRAWINGS

For a further understanding of the nature, objects, and advantages of the present invention, reference should be had to the following detailed description, read in conjunction with the following drawings, wherein like reference numerals denote like elements and wherein:
Figure I is a sectional elevational view of a user's hearing area to show the anatomy thereof;
Figure 2 is a sectional elevational view of a user's ear canal showing placement of a dam and mold material as part of the method of the present invention;
Figure 3 is a perspective view of the form portion used with the preferred method of the present invention;
Figure 4 is a perspective view illustrating shaping of the form as part of the method of the present invention;
Figure 5 is a perspective view illustrating a dipping of the form into a vessel carrying material for making the female mould as part of the method of the present invention;
Figure 6 is a perspective view illustrating a coating of the form with the female mould as part of the method of the present invention;
Figure 7 is a partial elevational view of the preferred embodiment of the apparatus of the present invention illustrating the mounting member and the plurality of the electronic hearing aid components;
Figure 7A is a cross-sectional view taken along the line 7A-7A in Figure 7;
Figure 7B is a partial view showing the portion indicated in Figure 7 as 7B;
Figure 8 is a elevational view of the lateral side of the mounting member taken along lines 8-8 of Figure 7;
Figure 9 is a perspective view illustrating the method step of joining the female mould to the mounting member at the medial side thereof;
Figure 10 is a perspective view of the preferred embodiment of the apparatus of the present invention and showing the method of the present invention after the joining of the female mould and mounting member;
Figure 11 is a perspective view illustrating the method step of adding filler material to the interior of the female mould and encapsulating electronic hearing aid component portions of the apparatus;
Figure 12 is a perspective view illustrating removal of the female mould after the filler material has set and encapsulating the electronic hearing aid components;
Figure 13 is a perspective of the preferred embodiment of the apparatus of the present invention and the method of the present invention illustrating removal of excess plate and tube material from the mounting member;
Figure 14 is a perspective view of the preferred embodiment of the apparatus of the present invention;
Figure 15 is an elevational view of the preferred embodiment of the apparatus of the present invention;
Figure 16 is an end view of the preferred embodiment of the apparatus of the present invention taken along lines 16-16 of Figure 15;
Figure 17 is a top view of the preferred embodiment of the apparatus of the present invention taken along lines 17-17 of Figure 15;
Figure 18 is a graphical representation of a comparison of real ear occlusion gain for the present invention versus a hard shell, hollow-type instrument; and
Figure 19 is a graphical representation showing a comparison of real ear aided gain obtained before acoustic feedback, comparing the present invention with a hard shell, hollow-type instrument.

### DETAILED DESCRIPTION OF THE INVENTION

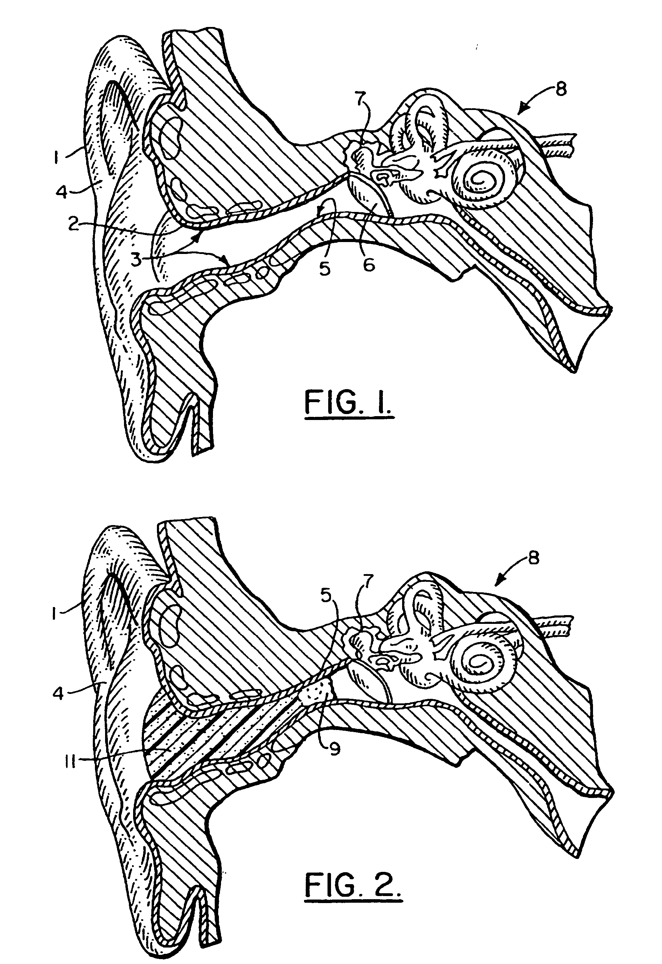

Figures 1 and 2 show a user's ear 1 and anatomical parts of the ear. In Figure 1 there can be seen the external auditory canal 2, ear canal wall 3, auricle 4, isthmus 5, tympanic membrane 6, middle ear 7 and inner ear 8. In Figure 2 a dam 9 such as a cotton dam or Otoblock® dam is positioned at the isthmus 5. The dam 9 is used as a first step of the method of the present invention wherein a form portion 11 or impression material is formed of silicone, methylmethacrylate or algenate. The form 11 is formed in between dam 9 and auricle 4 as shown in Figure 2.

During the method step of making the form 11, the form 11 conforms to all of the curvatures of the ear canal 3 so that an accurate form 11 is provided for making a female mould.

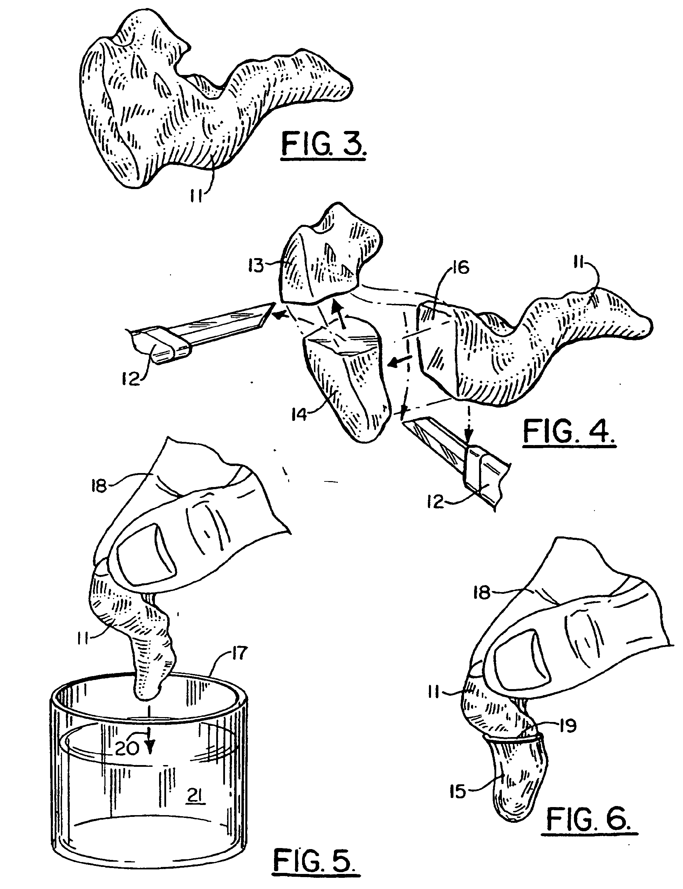

The female mould 15 is shown in Figures 5, 6 and 9-12. In Figures 3 and 4, the form 11 is shown after being removed from the ear 1 (Figure 3) and during a cutting of the form 11 using knives 12 to cut excess material that is designated as 13, 14 in Figure 4. The form 11 is separated from excess material 13 and 14 at sagittal plane 16. After the form 11 is trimmed in Figure 4, a technician's hand 18 dips the form 11 into vessel 17 as schematically indicated by the arrow 20. The vessel 17 includes a liquid material 21 that cures at room temperature such as room temperature curing methacrylate (sold by Esschem). It is preferable to use a clear material 21 in the method step shown in Figure 5.

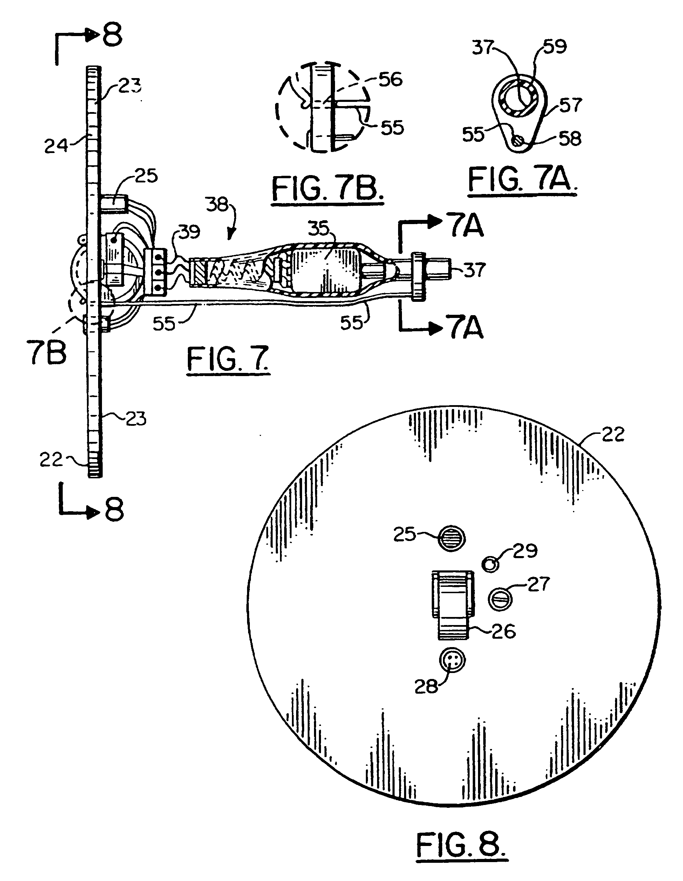

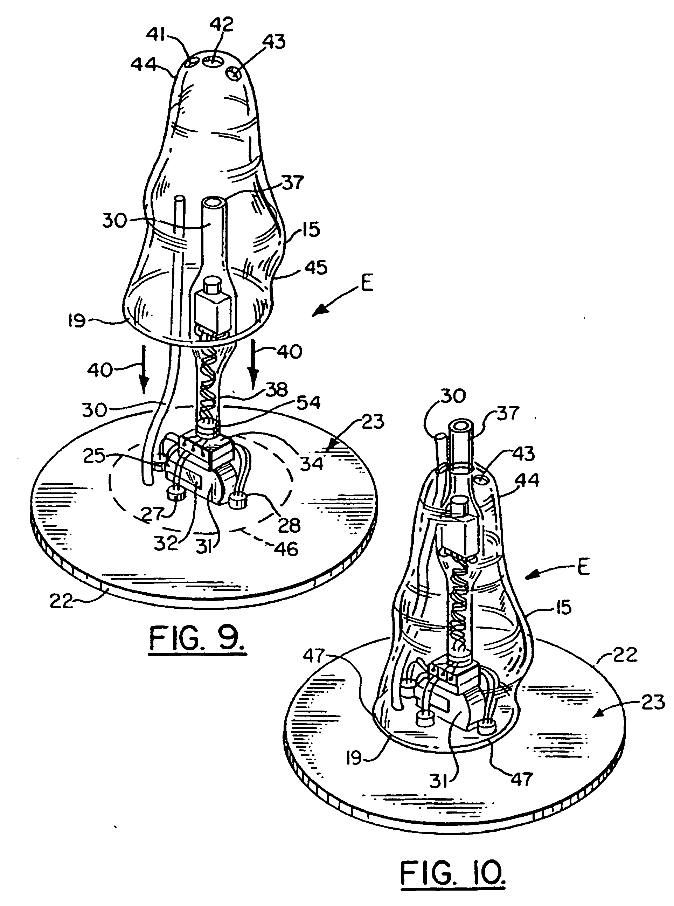

In Figure 6, the technician's hand 18 has removed the form 11 so that a coating of material 21 cures at room temperature (or with an ultraviolet light process) to form female mould 15 on form 11. After it cures, the female mould 15 is removed from form 11 for use as shown in Figures 9 and 10 during assembly of the apparatus 10 of the present invention. The mould 15 can be a few millimeters in wall thickness (typically 1-3 mm). A number of electronic components are mounted to a mounting member 22 prior to use of the female mould 15. Mounting member 22 provides a medial side 23 and lateral side 24. The medial side 23 supports a number of hearing aid electronic components as shown in Figures 7, 9, and 10. In Figure 7, these hearing aid electronic components include commercially available hearing aid components including a microphone 25, volume control, battery, socket or plug 28 for communicating with a computer, chip or micro processor circuit, wiring harness 38, input capacitor, amplifier 34, receiver/speaker 35, and receiver tube 37.

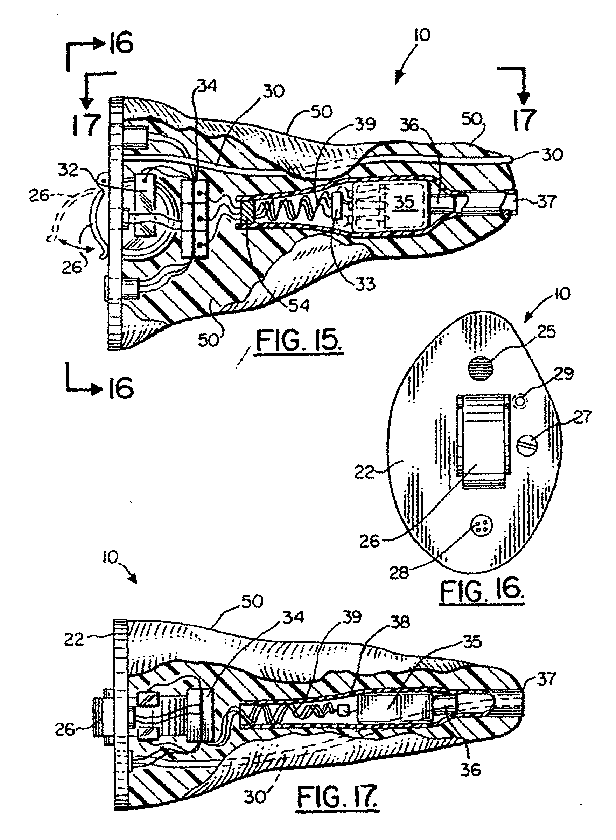

In Figure 8, the lateral side 24 of mounting member 22 shows the microphone 25, battery compartment 26, volume control 27, programming socket 28 for communicating with a computer, silicone plug 54 (see Figure 9), and vent opening 29 that communicates with vent tube 30 (see Figure 10). In Figure 9, battery 31 is shown housed in battery compartment 26. The electronic hearing aid components also include a battery terminal 32, voltage regulating capacitor 33 (see Figure 15), amplifier/microprocessor 34, receiver 35 having speaker port 36, and receiver tube 37. A wiring harness 38 includes a plurality of wires that connect to various electronic components of the hearing aid device together. The wiring harness 38 includes a length of wires 39 that are arranged in an S or multiple curved pattern as shown in Figure 7. This "S loop" configuration of wires 39 helps protect the integrity of the electronics when the hearing aid apparatus 10 is flexed as occurs during use because of its soft nature. Further, the S-loop wires 39 are preferably a 44 gauge five strand Litz wire (or magnet wire). The length of the S-loop wires 39 is preferably at least 1.5 times the distance between the terminals to the receiver (or microprocessor) 35 and the amplifier 34 terminals. These "S-Loop" wires 39 prevent excess tension or compression from being transmitted to the electronics during use (e.g. flexing, elongation, compression of hearing aid 10).

Vent tube 30 is anchored to the mounting member 22 and preferably also to one of the electronic components at a position spaced away from the mounting member 22. Vent tube 30 acts as a tensile load carrying member that carries tension so that the wiring harness 38 is substantially free of a tensile load that could damage the wiring harness 38. Also, when vent tube 30 is anchored to one of the electronic components (such as receiver 35) at a position spaced away from the mounting member 22, it may provide enough strain relief that it would not be necessary to coil wires 39 as shown (they could be straight instead).

Something else could be used as a load carrying member, in place of vent tube 30 (in which case vent tube 30 would not necessarily be anchored to one of the electronic components (such as receiver 35)) at a position spaced away from the mounting member 22. For example, a monofilament cantilever 55 can be used to carry tension so that tension is not transmitted to wiring harness 38. In Figures 7, 7A, and 7B the link 55 is anchored to plate 22 at opening 56. Fastener 57 affixes to receiver tube 37 at large opening 59. Monofilament cantilever 55 attaches to fastener 57 at smaller diameter opening 58. Alternatively, vent tube 30 could be manufactured of a tensile material that carries tensile load. The vent tube 30 would then be anchored to plate 22 and fastener 57 as the tensile member.

The monofilament cantilever 55 provides longitudinal stability to the body. It minimizes longitudinal displacement (stretching as well as compression) and thus acts as a longitudinal stabilizer (a longitudinal load carrying member).

After the electronic components (sometimes designated generally in the drawings by the letter "E") are assembled to the medial 23 side of mounting member 22, female mould 15 is used to complete the method of construction of the present invention as shown in Figure 9-13. In Figure 9, the female mould 15 is placed over the electronic components "E" beginning with the distal end portion of receiver tube 37 and the distal end portion of vent tube 30 as indicated by arrows 40 in Figure 9. A plurality of three openings 41, 42, 43 are provided at distal end 44 of female mould 15 as shown in Figure 9. The proximal end 45 of female mould 15 provides an annular edge surface 19 that engages the medial 23 side of mounting member 22 as indicated by the dotted line 46 in Figure 9.

A joint is formed between annular edge surface 19 of female mould 15 and medial surface 23 of mounting member 22 at a position schematically indicated as dotted line 46 in Figure 9, using the method of the present invention. The medial surface 23 of mounting member 22 is cleaned with a suitable solvent. Acetone can be used as a solvent in the case of a mounting plate 22 that is made of acrylic. The medial surface 23 of mounting member 22 is then painted with a primer using a swab or brush. The primer is allowed to dry. A bonding agent is then applied to the medial surface 23 of mounting member 22 and allowed to dry. The bonding agent or bonding enhancer can be product A-320 of Factor II, Inc. of Lakeside, Arizona, which is a member of the chemical family "silicone primer".

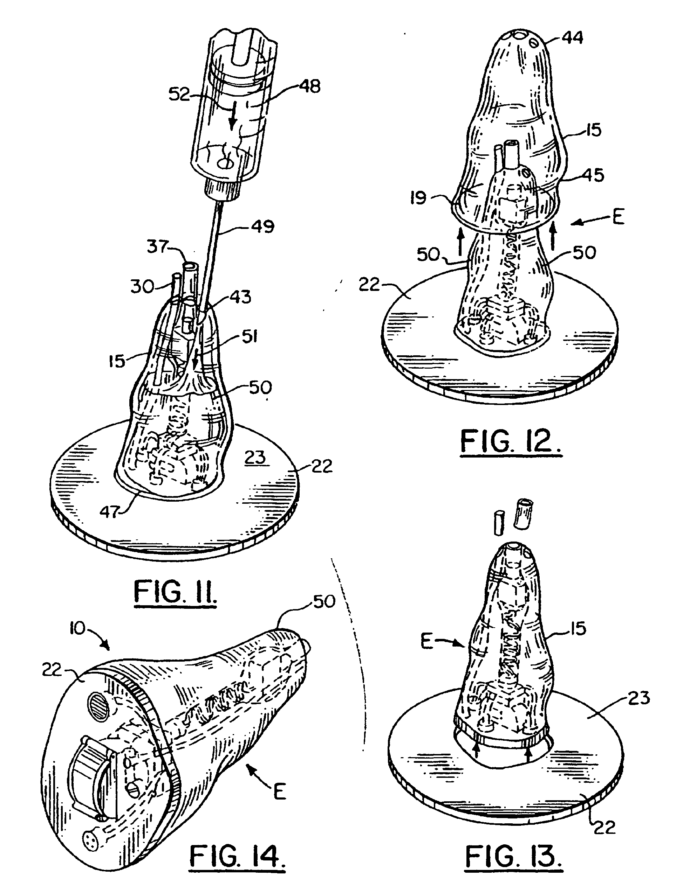

The female mould 15 is placed against the medial side 23 of mounting member 22. A liquid acrylic is used to form an acrylic seam at the interface of annular edge surface 19 of female mould 15 and the medial side 23 of mounting member 22 (see Figure 10). As the female mould 15 is assembled to mounting member 22, vent tube 30 passes through opening 41. Receiver tube 37 passes through opening 42. The opening 43 is then used for injection of filler material 50 (e.g. via needle 49) as shown by arrows 51, 52 in Figure 11. During this process, temporary seal 47 holds the liquid filler material 50 within the interior 53 that is formed by female mould 15 and mounting member 22. The filler material 50 can be a liquid during the injection step of Figure 11 so that it encapsulates at least the receiver/speaker electronic component 35 and preferably other components as well.

In Figure 12, the female mould 15 is removed after the material 50 has set. The mounting member 22 (which can be in the form of a circular, generally flat face plate) is then cut at the phantom line 46 that basically tracks the periphery of female mould 15 at annular edge surface 19 at proximal end 45 thereof. This cutting of the unused, unneeded part of mounting member 22 is shown in Figure 13. Figures 14-17 show the completed apparatus 10 of the present invention.

The present invention provides a soft, yet solid hearing aid instrument that will provide a more appropriate environment for both the high fidelity performance of today's advanced circuitry and the dynamic ear canal.

The present invention teaches a soft construction of at least the distal portion of the apparatus 10 so that at least the receiver/speaker is encapsulated with the soft material 50. This construction results in a precise representation of the human ear canal, flex with jaw motion, and cushion for the embedded electronic components "E".

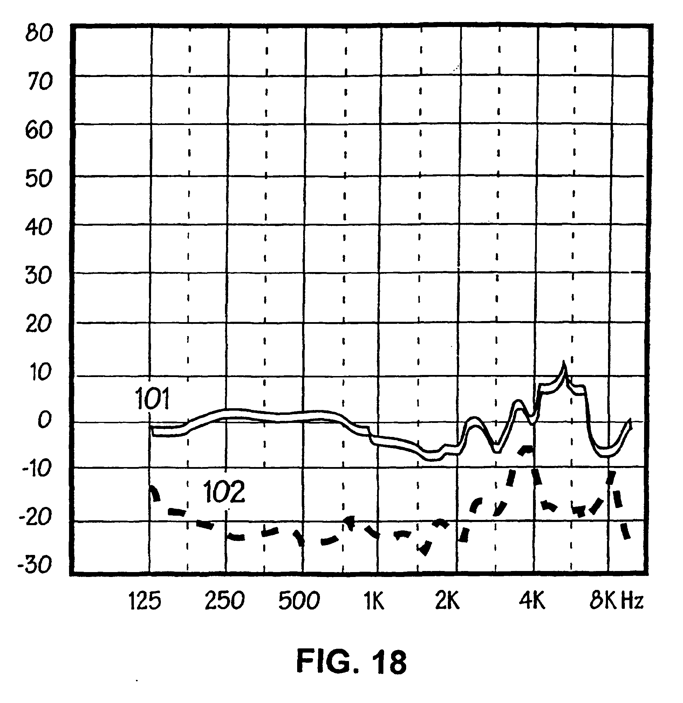

Figure 18 demonstrates real ear occlusion gain (REOG) finding obtained from a wearer having a tortuous ear canal. The curve 101 represents the REOG of a hard shell, hollow type hearing aid instrument. The curve 102 represents the REOG of an instrument 10 made according to the method of the present invention. As can be seen in Figure 18, the present invention instrument provided 20 dB more attenuation than did the hard shell, hallow hearing aid instrument represented by the curve 101. Because of the sharp first directional bend of the wearer's ear canal, the hard shell instrument could not be inserted without modification. The apparatus 10 of the present invention was insertable without modification thereby yielding a tighter seal in the wearer's ear.

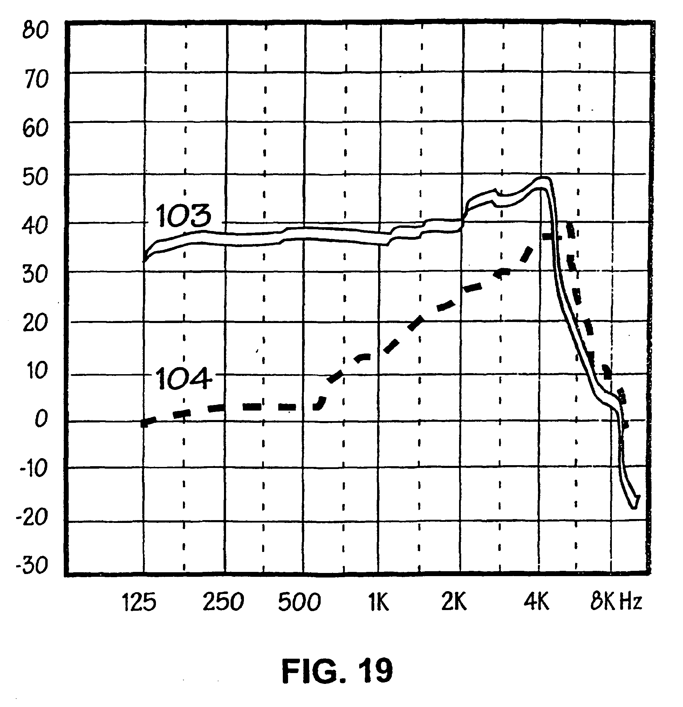

Figure 19 is a graphical representation that demonstrates real ear aided gain (REAG) findings obtained from a wearer having a tortuous ear canal. The curves shown (103, 104) were obtained from the instruments used to generate the finding shown in Figure 18. Curve 103 represent REAG before feedback of the apparatus 10 of the present invention. Curve 104 demonstrates the REAG before feedback of a hard shell, hollow type hearing aid instrument of the prior art. As can be seen in Figure 19, the instrument 10 of the present invention represented by curve 103 provided more gain across the frequencies. This REAG is inversely proportional to the amount of occlusion gain (REOG) or attenuation provided by the apparatus 10 of the present invention. It should be restated that, because of the sharp first directional bend of the wearer's ear canal, the hard shell, hollow type instrument of the prior art could not be inserted without being modified. The apparatus 10 of the present invention was insertable without modification, thus the present invention provides higher added gain values (REAG) when a more negative REOG can be achieved while maintaining comfort.

The apparatus 10 of the present invention will result in a better utilization of advanced circuitry and a more comfortable hearing instrument. The soft construction solves the problem of peripheral leakage, poor fit, and pivotal displacement that often occurs with jaw motion. Another problem that is solved with the present invention is the elimination of internal cross-talk of components housed in hollow shell type hearing aids.

Some preferred assembly and repair procedures follow:

### 1. SoftEar UVC3 casting procedure

To build this product correctly, we must insist that a thorough and complete impression with only minimal voids from otoblock lines be made. Those voids should be filled with wax, modeling clay, or another medium which will not add to the diameter of the canal. The length of the canal should, at the very least, extend beyond the second bend with the sound bore directed at the tympanic membrane.
I. The impression is cut to length as dictated by the desired style. Very little preparation should be done to the impression, and only then to slightly round off sharp edges that may cause discomfort to the wearer.
II. As a standard, the impression is dipped three times in pink wax kept at 200 _F (we have selected extra tough pink, dental base-plate wax manufactured by Hygenic).
III. That impression is invested in clear two-part silicone manufactured by Dreve. That two-part silicone is dispensed after having been blended in a Dreve dosper. The investment is typically cured at room temperature for approximately 10 minutes, but may be cured in a heated pressure pot at approximately 251b of pressure at 115_F for approximately 7 minutes. That step will ensure a minimal amount of air bubbles in the investment.
IV. UV material is poured into the investment (we are currently using Dreve as a UV chemical supplier). the material is cured for approximately 1.5 minutes (we are currently using a UVA Polylux 8 curing chamber manufactured by Dreve).
V. Following the first curing stage, excess UV material is recovered. The shell cavity is then filled with glysol solution, and cured in the UVA chamber for 4 minutes. The shell is removed from the silicone investment.
VI. The outside of the cast can be built up for structural stability, and is transferred to the electronics lab.

### 2. SoftEar UV-Exact casting procedure

This is procedure will result in absolute fidelity to the ear canal impression. All features of the impression will be in evidence in the final product.

To build this product correctly, we must insist that a thorough and complete impression with only minimal voids from otoblock lines be made. Those voids should be filled with wax, modeling clay, or another medium which will not add to the diameter of the canal. The length of the canal should, at the very least, extend beyond the second bend with the sound bore directed at the tympanic membrane.
I. The impression is cut to length as dictated by the desired style.
II. The impression is sprayed with a mold-release agent (we have selected Ease Release 200 manufactured by Mann Formulated Products).
III. The sprayed impression is dipped in photoplast gel (we have selected Keystone Gel # TG-71. Fotoplast gel from Dreve can be substituted if need be.)
IV. The coated impression is placed in the UVA curing chamber and is cured for approximately 3.5 minutes.
V. The impression is removed from the cast
VI. The cast is cleared of any debris, is placed in a glysol bath, and cured in the UVA chamber for 4 minutes.
VII. The cast is removed from the UVA chamber. Holes are then drilled for the receiver tube and for injection of the material. Keep in mind that the "injecting holes" should be drilled in areas which allow for filling of the cast without the trapping of air bubbles. The cast is then passed to electronics for loading of the components.

### 3. SoftEar preferred method of assembly and stabilization:

While any type of circuit can be used with the SoftEar procedure, our preferred procedure involves digitally programmable circuitry.
I. Into the top eye of the stabilizer fitting, an length of 30# test monofilament, which serves as the cantilever arm, is installed.
II. Onto the desired receiver, receiver wires are attached to the pads. A full boot is installed, leaving approximately 2 mm extended over the solder pads and leads.
III. Into the 2 mm extension, wire is folded over and tamped in to form an "s loop" which serves as a strain relief system. The end of the extension is then sealed with a silicone plug. Along the outside of the receiver boot, a small bead of silicone is installed on each side of the receiver which facilitates centering the receiver in the cast during the filling procedure.
IV. Onto the port of the receiver, receiver tubing is glued. The receiver tubing is of a diameter to fit through the bottom eye of the stabilizer fitting.
V. The receiver wires, the microphone wires (to which the microphone is not yet attached), and - if needed- the volume control wires are attached to the hybrid.
VI. Onto a standard faceplate, hard terminal wires are installed.
VII. A hard boot is installed over the battery contacts. If the boot has open sides, tape is applied to the outside of the boot, and fixed with cyanoacrylate.
VIII. On the faceplate the holes for the microphone and the programming socket are drilled, and a programming socket is installed into the faceplate.
IX. A jig with the dimensions of the hybrid to be used is employed to determine the optimum circuit location (determined by the cast supplied by the shell lab).
X. The jig is replaced with the hybrid, which is attached to the desired location. To provide extra strain relief, the receiver wires are routed below the hybrid contacts and are secured to either the hybrid or the battery boot. The programming socket is wired to the hybrid.
XI. Into the microphone hole, a grommet is installed and the microphone wires are pulled through the grommet. The grommet is then sealed with silicone.
XII. The hole for the cantilever assembly is then drilled into the faceplate.
XIII. The receiver tube is pulled through the bottom eye of the stabilizer fitting.
XIV. The cantilever arm is pulled through the hole in the faceplate. The cantilever arm is not secured at this point.
XV. The assembly is then inserted into the cast. The cantilever arm is manipulated until the receiver is at the desired site in the cast. The receiver is not secured at this time. The cantilever arm extending from the lateral side of the faceplate is clamped. The cast is removed from the assembly. The cantilever arm is cut flush with the outside of the faceplate. The arm is pushed through to the medial side of the faceplate, the tip of the arm is melted, and cyanoacrylate is applied to that area. The arm is reinserted into the faceplate so that it is flush with the lateral side of the faceplate.
XVI. The vent hole is drilled into the faceplate.
XVII. The assembly is then returned to the shell lab to be prepped for mounting.

### 4A. SoftEar faceplate preparation for closing

I. Acetone is painted onto the faceplate, within the candled areas on the medial side. Care is taken to not directly apply acetone to the wires or the components. This is allowed to dry for about 3 minutes.
II. The Primer is then added to the area that was previously covered with acetone. Due to the importance of this material in the bonding process, the Primer must be allowed to dry for 30 minutes.
III. Bonding Enhancer is then applied ,and allowed to dry for approximately 20 minutes.

### 4B.SoftEar closing procedure:(1)

I. The vent tubing is placed inside the cast.
II. The electronics are inserted into the cast, the receiver tube is pulled through the receiver tip, and the vent tube is pulled through the faceplate. The extended receiver tube is plugged with silicone to prevent contamination during the filling and curing process.
III. The prepared plates are then married to the cast by using a mix of cyanoacrylate and polymer. Ensure that the polymer bond is thick enough to prevent seepage under the seam and compromise of the bond at the outer edge of the product.
IV. The unit is then prepared for filling.

### 5. SoftEar filling procedure

I. The elastomer is blended in a 1:1 ratio. As a rule, about 7 grams of material are required for each instrument. If the mixture is too thick, functional fluid can be added to thin the blend, but the functional fluid should not exceed more than 10% of the total mixture..
II. The blended elastomer is subjected to 20 pounds of pressure for 2 minutes. The mix is then placed in an evacuator for five minutes. The mixture is again placed under 20 pounds of pressure for 2 more minutes. The result should be a bubble-free material.
III. The material is drawn into a syringe for injection into the cast.
IV. When filling the cast, best results are obtained by filling from the injection port near the plate, allowing the material to flow to the tip of the canal. Any bubbles trapped during the process can be forced out through the other ports.
V. Once the cast is filled, a final check of the receiver and microphone ports is done to ensure they are completely water tight. The filled instrument is placed in a water bath and kept at 110°F for 1 hour. Care should be taken to prevent the temperature from exceeding 140°F to avoid damaging the electronic components.

### 6. SoftEar final finish procedure

### Removing the instrument from the cast

I. The cured instrument is removed from the water bath. The cast is scored along its length with a metal burr.
II. Snips are used to cut along the score lines, and the pieces of the cast are removed from the elastomer body.
III. The faceplate is finished in a traditional manner.
IV. The instrument is then ready for mounting the microphone.

### Mounting the microphone

I. The grommet and the silicone plug are removed from the mic port. The mic wires are cut to the point that they extend 4 mm from the faceplate.
II. A windscreen is installed onto the mic. The mic wires are soldered onto the pads. The pads are insulated with a piece of Ad-Hear that has been cut to fit.
III. The mic is mounted into the port in the faceplate. The mic is stabilized in place by sealing the windscreen to the faceplate with monomer.

### Pre-QA preparation of the instrument.

I. The receiver tube and the real ear tube are cut to the point that they extend 2 mm from the receiver tip.
II. Following ANSI standards, the instrument is assessed to rule out internal feedback or other electroacoustic anomalies. If anomalies are present, the instrument is returned to the technician.
III. If the circuit used is a programmable unit, the instrument is interfaced with the programming instrumentation. The instrument is programmed to achieve desired parameters (we have chosen Fig. 6).
IV. The finish of the instrument is assessed. If there are elastomer nodules or any other faults, the instrument is returned to the shell lab.
V. If the instrument meets pre-QA criteria, the instrument is sent to QA.

### 7. SoftEar Recommended QA procedure

### Electronics:

I. Ensure that the Production Information slip accompanies the order in duplicate. If not, return it to Shell Lab.
II. Do not cut any tubing extending out of the faceplate. This is especially true of the real ear tubing whether it is fed through the vent tubing or is used as the vent tubing itself. At the receiver tip, let the real ear tube extend by 2mm whenever possible.
III. Ensure that the instrument has a serial number which agrees with the picking ticket. If it does not, return it to Shell Lab.
IV. If a programmable circuit has been used, ensure that the instrument has been programmed by an audiologist. If not, return the instrument to Electronics Lab.
V. Ensure that the investment and the impression is with the instrument. If not, return it to Shell Lab.
VI. As with normal QA procedure, if any electronic anomaly is present, return the instrument to Electronics Lab.
VII. If the instrument meets Electronics QA criteria, pass it on to Final QA.

### SoftEar recommended QA procedure

### Final QA:

I. Examine the instrument carefully for imperfections. Any detection of holes, separation at the faceplate, or cuts in the finish should result in returning the instrument to Shell Lab.
II. Examine the receiver tip. If Tygon tubing has been used, clip the receiver tubing so that it is recessed 1 mm from the receiver tip surface. If doing so will result in contacting the receiver port itself, consult with the Production Manager.
III. Whenever possible, let the real ear tubing extend by 2mm.
IV. Ensure that the receiver tip is smooth, and free of any elastomer nodules. If not, return the instrument to Shell Lab.
V. Ensure that the serial number, the investment, and the impression are present. In addition, ensure that the Product Information sheet is present in duplicate. If not, return it to Shell Lab.
VI. During the listening check, follow normal QA procedures. If any failure is detected, return it to Electronics Lab.
VII. If the instrument meets Final QA criteria, pass it on to Shipping.

### SoftEar recommended Shipping procedure

### Shipping:

I. Ensure that all paper work is in order. Make certain that one copy of the Product Information sheet accompanies the GHI file copy, and one copy accompanies the paperwork going to the customer.
II. Ensure that the investment and the impression are with the instrument. Return the investment and the impression to the designated SoftEar storage area.
III. Follow normal Shipping procedures.

### 8. SoftEar electronic repair

### Microphone repair

IV. If the FG-3329 mic has been used, the windscreen is removed and the mic is pulled free of the faceplate, and the wires are separated from the mic.
V. A new mic/windscreen assembly is attached to the wires, and the pads are insulated.
VI. The new mic is inserted into the mic port, and the windscreen is sealed to the faceplate with monomer.

### Receiver repair

I. A small cut is made into the elastomer perpendicular to the length of the receiver at the site of the receiver pads.
II. The receiver is removed from the body of the instrument through that cut, and the wires are separated from the receiver. It should be noted that the receiver tubing will stay in place.
III. The new receiver wires are attached to the receiver. A tubing spreader is used to distend the small cut from which the old receiver was removed.
IV. The port end of the receiver is inserted into the distended cut, and is guided into the receiver tubing.
V. Once the pad end of the receiver has been returned to the desired sit, the instrument is sent to the Shell Lab for sealing.

### 9. SoftEar body repair

### Repair body following electronic repair

I. The slit is treated with primer, which is allowed to dry for 3 minutes.
II. The slit is then treated with bonding enhancer, which is allowed to dry for 3 minutes.
III. The slit is filled with elastomer blend, ensuring that no bubbles infiltrate the mixture. Any excess material can be stored in the syringe under refrigeration for several days.
IV. The instrument is placed in a convection oven for 30 minutes at 60_C (140_F).
V. Any excess elastomer can be removed using a ruby studded bit.

### Modification of body for better fit

I. A new cast is formed from a fresh impression using the casting technique desired. The tip of the new cast is removed to serve as the primary filling hole. An over-fill hole should be drilled at the area of the aperture.
II. The elastomer is pared away from the receiver tip toward, but not including, the faceplate. Care should be taken to ensure that all internal components remain embedded in elastomer.
III. The trimmed instrument surface is coated with primer which is allowed to dry for 3 minutes.
IV. The primed instrument is then placed into the new cast. Care is taken to ensure correct alignment of components, and good conformity to the faceplate.
V. A syringe is filled with freshly blended elastomer. The elastomer is injected from the receiver end of the cast. Care should be taken to ensure the cast is completely filled, and that the elastomer is free of bubbles. Elastomer should exude through the over-fill hole.
VI. The recast instrument is placed in a convection oven set at 60_C (140_F), and allowed to cure for 1 hour.
VII. The cast is removed in the same manner as with a new instrument, and normal SoftEar finishing techniques are employed. The following table lists the parts numbers and parts descriptions as used herein and in the drawings attached hereto.

### PARTS LIST

| Part Number | Description |
|---|---|
| 1 | ear |
| 2 | external auditory canal |
| 3 | ear canal wall |
| 4 | auricle |
| 5 | isthmus |
| 6 | tympanic membrane |
| 7 | middle ear |
| 8 | inner ear |
| 9 | dam |
| 10 | hearing aid |
| 11 | form |
| 12 | knife |
| 13 | excess material |
| 14 | excess material |
| 15 | female mold |
| 16 | sagittal plane |
| 17 | vessel |
| 18 | technician's fingers |
| 19 | annular surface |
| 20 | arrow |
| 21 | mold material |
| 22 | mounting member |
| 23 | medial side |
| 24 | lateral side |
| 25 | microphone |
| 26 | battery compartment |
| 27 | volume control |
| 28 | programming socket |
| 29 | vent opening |
| 30 | vent tube |
| 31 | battery |
| 32 | battery terminal |
| 33 | voltage regulating capacitor |
| 34 | amplifier/microprocessor |
| 35 | receiver |
| 36 | receiver port |
| 37 | receiver tube |
| 38 | wiring harness |
| 39 | s-loop wires |
| 40 | arrow |
| 41 | opening |
| 42 | opening |
| 43 | opening |
| 44 | distal end |
| 45 | proximal end |
| 46 | dotted line |
| 47 | temporary seal |
| 48 | syringe |
| 49 | needle |
| 50 | filler material |
| 51 | arrow |
| 52 | arrow |
| 53 | interior space |
| 54 | silicone plug |
| 55 | monofiament cantilever |
| 56 | opening |
| 57 | fastener |
| 58 | small opening |
| 59 | large opening |

The foregoing embodiments are presented by way of example only; the scope of the present invention is to be limited only by the following claims.

## Claims

1. An in the ear hearing aid comprising
a plate member (22) with electronic hearing aid components mounted thereto;
and **characterised in** further comprising
a soft polymeric body (50) that is bonded to the plate member (22), which encapsulates at least some of the electronic hearing aid components and is shaped to conform to the ear canal (2) of a user;
whereby the soft polymeric body (50) and encapsulated electronic hearing aid components define a soft structure that is compliant to the ear canal (2) during use, is substantially solid and free of void spaces between said at least some of the electronic components and the ear canal (2);
and wherein the combination of the soft polymeric body (50) and encapsulated electronic hearing aid components minimizes feedback.

2. A hearing aid as claimed in claim 1 wherein the plate member (22) has opposing generally flat sides including a medial side (23) and a lateral side (24), and the electronic hearing components are mounted to extend medially from the medial side (23).

3. A hearing aid as claimed in claim 2 wherein the plate member (22) carries a number of controls for the electronic components on the lateral side (24) of the plate member (22).

4. A hearing aid as claimed in any preceding claim wherein the plate member (22) is generally circular in shape.

5. A hearing aid as claimed in any preceding claim wherein a bonding enhancer forms an interface between the soft polymeric body (50) and the plate member (22).

6. A hearing aid as claimed in any preceding claim wherein the plate member (22) is much harder than the soft polymeric body (50).

7. A hearing aid as claimed in claim 6 wherein the plate member (22) is a rigid plastic member.

8. A hearing aid as claimed in either claim 6 or 7 wherein the plate member (22) is an acrylic member.

9. A hearing aid as claimed in any preceding claim wherein the electronic hearing aid components include a receiver (35).

10. A hearing aid as claimed in any preceding claim wherein the electronic hearing aid components include a printed circuit board.

11. A hearing aid as claimed in any preceding claim wherein the electronic hearing aid components include a microprocessor.

12. A hearing aid as claimed in any preceding claim wherein the electronic hearing aid components include a wiring harness (38) that is configured to maintain its integrity during flexing of the soft compliant structure.

13. A hearing aid as claimed in claim 12 wherein the wiring harness (38) includes a plurality of wires that extend between two further electronic hearing components.

14. A hearing aid as claimed in either claim 12 or 13 wherein the wiring harness (38) is a multiple S-loop wiring harness (39).

15. A hearing aid as claimed in any of claims 12 to 14 wherein the electronic hearing aid components include a load carrying member for preventing at least some transfer of tensile load to the wiring harness (38).

16. A hearing aid as claimed in claim 15 wherein the load carrying member provides longitudinal stability.

17. A hearing aid as claimed in either claim 15 or 16 wherein the load carrying member is a vent tube (30)

18. A hearing aid as claimed in any preceding claim sized to fit completely in the ear canal (2) of a user.

19. A hearing aid as claimed in any preceding claim wherein one or more electronic hearing aid components are positioned next to the isthmus (5) of the user's ear canal (2) during use.

20. A hearing aid as claimed in any preceding claim wherein one or more electronic hearing aid components are positioned to direct amplified sound to the tympanic membrane (6) of the user's ear (1) during use.

21. A hearing aid as claimed in any preceding claim wherein the soft polymeric body has a hardness of between about 3 and 55 Durometer Shore A.

22. A method of manufacturing a hearing aid **characterised in** comprising the steps of:
(a) forming a hollow shell (15) with an inside surface that approximates to the shape of the human ear canal (2), the shell (15) being of a soft polymeric material;
(b) providing a mounting member (22);
(c) mounting electronic hearing aid components to the mounting member (22);
(d) temporarily joining the hollow shell (15) to the mounting member (22) to define a mould cavity;
(e) filling the mould cavity with a soft polymeric material (50) that substantially encapsulates the electronic components;
(f) eliminating substantially all void space between the shell (15) and the electronic components with the filling (50) in step "e".
(g) allowing the soft polymeric material (50) to cure;
(h) removing the shell (15);
(i) wherein in step "e" the combination of the electronic components and fill material (50) define a soft structure that is compliant to ear canal (2) movement during use, is substantially solid and free from void spaces between at least some of the electronic components and the ear canal (2).

23. A method according to claim 22 further comprising the step of making an impression of the user's ear canal (2) to construct a form (11) and using the form (11) to shape the inside surface of the hollow shell (15).

24. A method according to claim 22 or 23 further comprising the step of using a form (11) to shape the inside surface of the hollow shell (15).

25. A method according to any of claims 22 to 24 wherein step "b" comprises providing a mounting member (22) that is rigid plastic member.

26. A method according to any of claims 22 to 25 wherein step "b" comprises providing a mounting member (22) that is an acrylic member.

27. A method according to any of claims 22 to 26 wherein in step "b" the mounting member (22) has medial and lateral side portions (23, 24).

28. A method according to claim 27 wherein in step "c" electronic hearing aid components are attached to the medial side portions (23) of the mounting member (22).

29. A method according to any one of claims 22 to 28 wherein the shell (15) and mounting member (22) are temporarily joined with a seal in step "d".

30. A method according to any one of claims 22 to 29 wherein in step "e" at least a receiver (35) is encapsulated.

31. A method according to any one of claims 22 to 30 wherein in step "e" at least a receiver (35) and wiring harness (38) are encapsulated.

32. A method according to any one of claims 22 to 31 wherein step "e" comprises filling the shell (15) with a soft silicone material.

33. A method according to any one of claims 22 to 32 wherein in step "e" the soft polymeric material (50) has a hardness of between about 3 and 55 Durometer Shore A.

34. A method according to any of claims 22 to 33 further comprising the step of using a bonding enhancer to join the soft polymeric material (50) to the mounting member (22)

35. A method according to claim 34 further comprising the step of applying multiple coatings of the bonding enhancer to the mounting member (22).

36. A method according to any of claims 22 to 35 wherein the hearing aid is sized to fit completely in the ear canal (2) of a user.

## Patentansprüche

1. Im Ohr tragbare Hörhilfe mit:
einem Plattenglied (22) mit elektronischen Hörhilfekomponenten, die daran befestigt sind;
die **dadurch gekennzeichnet ist, dass** sie des Weiteren aufweist:
einen weichen Polymerkörper (50), der mit dem Plattenglied (22) verbunden ist, der zumindest einige der elektronischen Hörhilfekomponenten einkapselt und der geformt ist, um sich dem Gehörgang (2) eines Benutzers anzupassen;
wobei der weiche Polymerkörper (50) und die eingekapselten elektronischen Hörhilfekomponenten eine weiche Struktur definieren, die während einer Verwendung gegenüber dem Ohrkanal (2) nachgiebig ist und die im Wesentlichen fest und frei von Hohlräumen zwischen zumindest einigen der elektronischen Komponenten und dem Ohrkanal (2) ist;
und wobei die Kombination des weichen Polymerkörpers (50) und der eingekapselten elektronischen Hörhilfekomponenten eine Rückkopplung minimiert.

2. Hörhilfe nach Anspruch 1, wobei das Plattenglied (22) sich gegenüberliegende, im Allgemeinen ebene Seiten aufweist, einschließlich einer mittleren Seite (23) und einer seitlichen Seite (24), und wobei die elektronischen Hörkomponenten befestigt sind, um sich mittig von der mittleren Seite (23) zu erstrecken.

3. Hörhilfe nach Anspruch 2, wobei das Plattenglied (22) eine Anzahl von Steuerungen für die elektronischen Komponenten auf der lateralen Seite (24) des Plattenglieds (22) trägt.

4. Hörhilfe nach einem der vorhergehenden Ansprüche, wobei das Plattenglied (22) im Allgemeinen kreisförmig ist.

5. Hörhilfe nach einem der vorhergehenden Ansprüche, wobei ein Bindeverstärker eine Schnittstelle zwischen dem weichen Polymerkörper (50) und dem Plattenglied (22) bildet.

6. Hörhilfe nach einem der vorhergehenden Ansprüche, wobei das Plattenglied (22) viel härter als der weiche Polymerkörper (50) ist.

7. Hörhilfe nach Anspruch 6, wobei das Plattenglied (22) ein steifes Plastikglied ist.

8. Hörhilfe nach Anspruch 6 oder 7, wobei das Plattenglied (22) ein Acrylglied ist.

9. Hörhilfe nach einem der vorhergehenden Ansprüche, wobei die elektronischen Hörhilfekomponenten einen Empfänger (35) umfassen.

10. Hörhilfe nach einem der vorhergehenden Ansprüche, wobei die elektronischen Hörhilfekomponenten eine gedruckte Leiterplatte umfassen.

11. Hörhilfe nach einem der vorhergehenden Ansprüche, wobei die elektronischen Hörhilfekomponenten einen Mikroprozessor umfassen.

12. Hörhilfe nach einem der vorhergehenden Ansprüche, wobei die elektronischen Hörhilfekomponenten einen Kabelbaum (38) umfassen, der konfiguriert ist, um seine Unversehrtheit während einem Biegen der weichen, nachgiebigen Struktur beizubehalten.

13. Hörhilfe nach Anspruch 12, wobei der Kabelbaum (38) eine Vielzahl von Drähten umfasst, die sich zwischen zwei weiteren elektronischen Hörkomponenten erstrecken.

14. Hörhilfe nach Anspruch 12 oder 13, wobei der Kabelbaum (38) ein Kabelbaum (39) aus mehren S-Schleifen ist.

15. Hörhilfe nach einem der Ansprüche 12 bis 14, wobei die elektronischen Hörhilfekomponenten ein Versteifungsglied zum Verhindern von zumindest einer teilweisen Übertragung einer Dehnungsbelastung an den Kabelbaum (38) umfassen.

16. Hörhilfe nach Anspruch 15, wobei das Versteifungsglied eine longitudinale Stabilität vorsieht.

17. Hörhilfe nach Anspruch 15 oder 16, wobei das Versteifungsglied ein Belüftungsrohr (30) ist.

18. Hörhilfe nach einem der vorhergehenden Ansprüche, die dimensioniert ist, um vollständig in den Ohrkanal (2) des Benutzers zu passen.

19. Hörhilfe nach einem der vorhergehenden Ansprüche, wobei eine oder mehrere elektronische Hörhilfekomponenten während einer Verwendung neben dem Isthmus (5) des Gehörgangs (2) des Benutzers angeordnet sind.

20. Hörhilfe nach einem der vorhergehenden Ansprüche, wobei eine oder mehrere elektronische Hörhilfekomponenten angeordnet sind, um ein Geräusch während einer Verwendung direkt an das Trommelfell (6) des Benutzerohrs (1) zu verstärken.

21. Hörhilfe nach einem der vorhergehenden Ansprüche, wobei der weiche Polymerkörper eine Härte zwischen ungefähr 3 und 55 Durometer Shore A aufweist.

22. Verfahren zum Herstellen einer Hörhilfe, **dadurch gekennzeichnet, dass** es die folgenden Schritte aufweist:
(a) Bilden einer hohlen Hülle (15) mit Innenfläche, die sich der Form des menschlichen Gehörgangs (2) annähert, wobei die Hülle (15) aus einem weichen Polymermaterial besteht;
(b) Vorsehen eines Befestigungsglieds (22);
(c) Befestigen von elektronischen Hörhilfekomponenten an dem Befestigungsglied (22),
(d) vorübergehendes Verbinden der hohlen Hülle (15) mit dem Befestigungsglied (22), um einen Gusshohlraum zu definieren;
(e) Füllen des Gusshohlraums mit einem weichen Polymermaterial (50), das im Wesentlichen die elektronischen Komponenten umkapselt;
(f) Eliminieren von im Wesentlichen jedem Hohlraum zwischen der Hülle (15) und den elektronischen Komponenten durch das Füllen (50) beim Schritt "e";
(g) das weiche Polymermaterial (50) aushärten lassen;
(h) Entfernen der Hülle (15);
(i) wobei beim Schritt "e" die Kombination der elektronischen Komponenten und des Füllmaterials (50) eine weiche Struktur definiert, die gegenüber einer Bewegung des Gehörgangs (2) während einer Verwendung nachgiebig ist und die im Wesentlichen fest und frei von Hohlräumen zwischen zumindest einigen der elektronischen Komponenten und dem Gehörgang (2) ist.

23. Verfahren nach Anspruch 22, das des Weiteren den Schritt aufweist: sich einen Eindruck machen von dem Gehörgang (2) des Benutzers, um eine Form (11) zu konstruieren, und Verwenden der Form (11), um die Innenfläche der hohlen Hülle (15) zu formen.

24. Verfahren nach Anspruch 22 oder 23, das des Weiteren den Schritt eines Verwendens einer Form (11) aufweist, um die Innenfläche der hohlen Hülle (15) zu formen.

25. Verfahren nach einem der Ansprüche 22 bis 24, wobei der Schritt "b" ein Vorsehen eines Befestigungsglieds (22) aufweist, welches ein steifes Plastikglied ist.

26. Verfahren nach einem der Ansprüche 22 bis 25, wobei der Schritt "b" ein Vorsehen eines Befestigungsglieds (22) aufweist, das ein Acrylglied ist.

27. Verfahren gemäß einem der Ansprüche 22 bis 26, wobei beim Schritt "b" das Befestigungsglied (22) einen mittleren Teil (23) und einen seitlichen Teil (24) aufweist.

28. Verfahren nach Anspruch 27, wobei beim Schritt "c" elektronische Hörhilfekomponenten an dem mittleren Seitenteil (23) des Befestigungsglieds (22) angebracht werden.

29. Verfahren nach einem der Ansprüche 22 bis 28, wobei die Hülle (15) und das Befestigungsglied (22) beim Schritt "d" vorübergehend mit einer Dichtung verbunden werden.

30. Verfahren nach einem der Ansprüche 22 bis 29, wobei beim Schritt "e" zumindest ein Empfänger (35) umkapselt wird.

31. Verfahren nach einem der Ansprüche 22 bis 30, wobei beim Schritt "e" zumindest ein Empfänger (35) und ein Kabelbaum (38) umkapselt werden.

32. Verfahren nach einem der Anspräche 22 bis 31, wobei der Schritt "e" ein Füllen der Hülle (15) mit einem weichen Silikonmaterial aufweist.

33. Verfahren nach einem der Ansprüche 22 bis 32, wobei beim Schritt "e" das weiche Polymermaterial (50) eine Härte zwischen ungefähr 3 und 55 Durometer Shore A aufweist.

34. Verfahren nach einem der Ansprüche 22 bis 23, das des Weiteren den Schritt eines Verwendens eines Bindeverstärkers aufweist, um das weiche Polymermaterial (50) mit dem Befestigungsglied (22) zu verbinden.

35. Verfahren nach Anspruch 34, das des Weiteren den Schritt eines Auftragens von mehreren Schichten des Bindeverstärkers auf das Befestigungsglied (22) aufweist.

36. Verfahren nach einem der Ansprüche 22 bis 35, wobei die Hörhilfe dimensioniert ist, um vollständig in den Gehörgang (2) eines Benutzers zu passen.

## Revendications

1. Prothèse auditive comprenant :
un élément en plaque (22) avec des composants électroniques de prothèse auditive, montés sur celui-ci ;
**caractérisée en ce qu'**elle comporte en outre :
un corps (50) polymère (50) lié à l'élément plaque (22) et qui encapsule au moins une partie des composants de prothèse électronique et est conformé au canal (2) auditif de l'utilisateur ; tandis que le corps (50) polymère souple et les composants de prothèse électronique encapsulés définissent une structure souple complémentaire du canal (2) auditif durant l'utilisation, et sensiblement solide et libre d'espaces vides entre lesdits au moins composants électroniques et le canal auditif (2) ; et tandis que la combinaison du corps polymérique souple (50) et les composants de prothèse électronique auditive encapsulés minimisent la rétroaction.

2. Prothèse auditive selon la revendication 1, **caractérisée en ce que** l'élément plaque (22) présente des côtés de forme générale plate et se faisant face, incluant un côté médian (23) et un côté latéral (24) et les composants auditifs électroniques sont montés pour s'étendre de façon médiane à partir du côté médian (23).

3. Prothèse auditive selon la revendication 2, **caractérisée en ce que** l'élément plaque (22) porte un certain nombre de contrôles pour les composants électroniques sur le côté latéral (20) de l'élément plaque (22).

4. Prothèse auditive selon l'une des revendications précédentes, **caractérisée en ce que** l'élément plaque (22) est de forme générale circulaire.

5. Prothèse auditive selon l'une des revendications précédentes, **caractérisée en ce qu'**un amplificateur de liaison forme une interface entre le corps polymérique souple (50) et l'élément plaque (22).

6. Prothèse auditive selon l'une des revendications précédentes, **caractérisée en ce que** l'élément plaque (22) est bien plus dur que le corps polymérique souple (50).

7. Prothèse auditive selon la revendication 6, **caractérisée en ce que** l'élément plaque (22) est un élément plastique rigide.

8. Prothèse auditive selon l'une des revendications 6 ou 7, **caractérisée en ce que** l'élément plaque (22) est un élément acrylique.

9. Prothèse auditive selon l'une des revendications précédentes, **caractérisée en ce que** les éléments de prothèse électronique incluent un récepteur (35).

10. Prothèse auditive selon l'une des revendications précédentes, **caractérisée en ce que** les composants de prothèse auditive électronique incluent un circuit imprimé.

11. Prothèse auditive selon l'une des revendications précédentes, **caractérisée en ce que** les composants de prothèse électronique incluent un microprocesseur.

12. Prothèse auditive selon l'une des revendications précédentes,
**caractérisée en ce que** les composants de prothèse électronique incluent des fils couplés (38) configurés pour maintenir son intégrité lors de la flexion de la structure conformant souple.

13. Prothèse auditive selon la revendication 12, **caractérisée en ce que** les fils couplés (38) présentent une pluralité de fils qui s'étendent entre deux composants auditifs électroniques.

14. Prothèse auditive selon l'une des revendications 12 ou 13, **caractérisée en ce que** les fils couplés (38) sont constitués d'un ensemble harnais filaire (39) à boucles multiples en forme de S.

15. Prothèse auditive selon l'une des revendications 12 à 14, **caractérisée en ce que** les composants de prothèse auditive électronique incluent un élément de portage de charges pour empêcher au moins une partie du transfert de la charge tensile des fils couplés.

16. Prothèse auditive selon la revendication 15, **caractérisée en ce que** l'élément de portage de charge procure une stabilité longitudinale.

17. Prothèse auditive selon l'une des revendications 15 ou 16, **caractérisée en ce que** l'élément porteur de charge est un tube de ventilation (30).

18. Prothèse auditive selon l'une des revendications précédentes, conformée de manière à s'adapter complètement au canal auditif (2) de l'utilisateur.

19. Prothèse auditive selon l'une des revendications précédentes,
**caractérisée en ce qu'**un ou plusieurs composants de prothèse auditive électronique sont disposés à proximité de l'isthme (5) du canal auditif de l'oreille de l'utilisateur durant l'utilisation.

20. Prothèse auditive selon l'une des revendications précédentes, **caractérisée en ce qu'**un ou plusieurs composants de prothèse auditive électronique sont positionnés pour amplifier de manière directe le son à la membrane du tympan (6) de l'oreille de l'utilisateur lors de l'utilisation.

21. Prothèse auditive selon l'une des revendications précédentes, **caractérisée en ce que** le corps polymérique souple présente une dureté entre 3 et 55 duromètre Shore A.

22. Procédé de fabrication de prothèse auditive **caractérisé en ce qu'**il comporte les étapes de :
(a) former une coquille (15) creuse avec une surface intérieure qui est conforme à la forme du canal auditif humain (2), la coquille (15) étant en matériau polymérique souple.
(b) réaliser un élément de montage (22)
(c) monter les composants auditifs de prothèse électronique sur l'élément de montage (22)
(d) temporairement joindre la coquille creuse (15) à l'élément de montage (22) pour définir une cavité de moulage
(e) remplir la cavité de moule avec un matériau polymérique (50) qui sensiblement encapsule les composants électroniques
(f) éliminer sensiblement tout espace vide entre la coquille (15) et les composants électroniques avec le remplissage (50) dans l'étape «e»
(g) permettre au matériau (50) polymérique souple de durcir
(h) enlever la coquille (15)
(i) **caractérisé en ce que** dans l'étape « e » la combinaison des composants électroniques et du matériau de remplissage définit une structure souple qui est conforme aux mouvements du canal auditif (2) durant l'utilisation et est sensiblement solide et libre de tout espace vide entre au moins une partie des composants électroniques et le canal auditif (2).

23. Procédé selon la revendication 22, **caractérisé en ce qu'**il comporte en outre l'étape de réaliser une impression du canal auditif (2) de l'utilisateur pour construire une forme (11) et utiliser la forme (11) pour conformer la surface intérieure de la coquille creuse (15).

24. Procédé selon la revendication 22 ou 23, **caractérisé en ce qu'**il comporte l'étape d'utiliser une forme (11) pour conformer la surface intérieure de la coquille (15) creuse.

25. Prothèse auditive selon l'une des revendications 22 à 24, **caractérisée en ce que** l'étape « b » comporte le fait de fournir un élément de montage (22) qui soit un élément en plastique rigide.

26. Prothèse auditive selon l'une des revendications 22 à 25, **caractérisée en ce que** l'étape « b » comporte le fait de fournir un élément de montage (22) qui est un élément en acrylique.

27. Prothèse auditive selon l'une des revendications 22 à 26, **caractérisée en ce que** dans l'étape « b », l'élément de montage (22) présente des parties de côtés latéral et médian (23, 24).

28. Prothèse auditive selon la revendication 27, **caractérisée en ce que** dans l' étape « c », les composants de prothèse auditive électronique sont fixés à la partie latérale médiane (23) de l'élément de montage (22).

29. Procédé selon l'une des revendications 22 à 28, **caractérisé en ce que** la coquille (15) et l'élément de montage (22) sont temporairement joints avec un scellement dans l'étape « d ».

30. Procédé selon l'une des revendications 22 à 29, **caractérisé en ce que** dans l'étape (e), au moins un récepteur (35) est encapsulé.

31. Procédé selon l'une des revendications 22 à 30, **caractérisé en ce que** dans l'étape « e », au moins un récepteur (35) et des fils couplés (38) sont encapsulés.

32. Procédé selon l'une des revendications 22 à 31, **caractérisé en ce que** l'étape "e" comporte le remplissage de la coquille (15) avec un matériau silicone souple.

33. Procédé selon l'une des revendications 22 à 31, **caractérisé en ce que** dans l'étape « e », le matériau (50) polymérique souple présente une dureté comprise entre environ 3 et 55 duromètre Short A.

34. Procédé selon l'une des revendications 22 à 33, **caractérisé en ce qu'**il comporte en outre l'étape d'utiliser un amplificateur de liaison pour joindre le matériau polymérique souple (50) à l'élément de montage (22).

35. Procédé selon la revendication 34, **caractérisé en ce qu'**il comporte l'étape d'appliquer des couches multiples sur l'amplificateur de liaison à l'élément de montage (22).

36. Procédé selon l'une des revendications 22 à 35, **caractérisé en ce que** la prothèse est conformée pour s'adapter complètement au canal auditif (2) de l'utilisateur.
